# EUROPEAN PATENT APPLICATION

(11) **EP 4 062 822 A2**
(43) Date of publication of application: **28.09.2022**
(21) Application number: 22162310.1
(22) Date of filing: 15.03.2022
(51) Int. Cl.: A61B 5/00, A61B 5/242, G16H 30/40

(54) **BIOMAGNETISM MEASUREMENT APPARATUS, BIOMAGNETISM MEASUREMENT SYSTEM, BIOMAGNETISM MEASUREMENT METHOD, AND BIOMAGNETISM MEASUREMENT PROGRAM**

(30) Priority: 22.03.2021 JP 2021047934; 12.12.2021 JP 2021196264
(71) Applicant: Ricoh Company, Ltd., Tokyo 143-8555 (JP)
(72) Inventor: Ishida, Koki, Tokyo, 143-8555 (JP); Watanabe, Taishi, Tokyo, 143-8555 (JP); Kawabata, Shigenori, Tokyo, 113-8510 (JP); Hashimoto, Jun, Tokyo, 113-8510 (JP)
(74) Representative: Marks & Clerk LLP

(57) **Abstract**

A biomagnetism measurement apparatus includes a region-of-interest setting section configured to set a curved surface as a region-of-interest based on a morphological image including a portion to be evaluated of a subject, the morphological image being acquired by an image acquisition section, and the curved surface being a surface on which an anterior-posterior position of the subject changes in a cranial-caudal direction and in a left-right direction along a position of a nerve to be evaluated, and an estimation section configured to estimate current distribution in the region-of-interest based on magnetic data, the magnetic data being acquired by measuring a magnetic field with a magnetic measurement section, and the magnetic field being generated by an electrical activity of a nerve in the portion to be evaluated.

## Description

### BACKGROUND OF THE INVENTION

### 1. Field of the Invention

The present invention relates to a biomagnetism measurement apparatus, a biomagnetism measurement system, a biomagnetism measurement method, and a biomagnetism measurement program.

### 2. Description of the Related Art

Biomagnetism measurement apparatus for measuring magnetism from the neck, lumbar, or the like, includes, for example, a sensor array comprising a plurality of magnetic sensors arranged vertically and horizontally, and a marker positioned in proximity to the sensor array and which is difficult for X-rays to pass through. In the measurement of biomagnetism, a positional relationship between the sensor array and a nerve to be measured is acquired by taking an X-ray image of a subject and the marker from the side of the sensor array (see Patent Document 1).

Patent Document 1: JP Patent No. 4834076

The present disclosure has an object to accurately estimate the nerve activity current outside the spinal canal together with the nerve activity current of the spinal cord.

### SUMMARY OF THE INVENTION

A biomagnetism measurement apparatus, according to an embodiment of the present disclosure, includes a region-of-interest setting section configured to set a curved surface as a region-of-interest based on a morphological image including a portion to be evaluated of a subject, the morphological image being acquired by an image acquisition section, and the curved surface being a surface on which an anterior-posterior position of the subject changes in a cranial-caudal direction and in a left-right direction along a position of a nerve to be evaluated, and an estimation section configured to estimate current distribution in the region-of-interest based on magnetic data, the magnetic data being acquired by measuring a magnetic field with a magnetic measurement section, and the magnetic field being generated by an electrical activity of a nerve in the portion to be evaluated.

The nerve activity current outside the spinal canal together with the nerve activity current of the spinal cord can be estimated accurately.

### BRIEF DESCRIPTION OF THE DRAWINGS

FIG. 1 is a configuration diagram illustrating an example of a biomagnetism measurement system including a biomagnetism measurement apparatus according to a first embodiment of the present invention;
FIG. 2 is a flow diagram illustrating an example of an operation of the biomagnetism measurement apparatus of FIG. 1;
FIG. 3 is a diagram illustrating an example of a morphological image of a subject acquired by a morphological image acquisition unit of FIG.1;
FIG. 4 is a diagram illustrating an example of a region-of-interest set by a region-of-interest setting unit of FIG.1;
FIG. 5 is a perspective view illustrating a reference region-of-interest of FIG.4 and a region-of-interest that has been reset;
FIG. 6 is a diagram illustrating an example of a current distribution in the region-of-interest estimated based on magnetic data and a change in a current waveform in a virtual electrode set in the region-of-interest;
FIG. 7 is a diagram illustrating another example of a morphological image of the subject acquired by the morphological image acquisition section of FIG. 1;
FIG. 8 is a diagram illustrating another example of a current distribution in the region-of-interest estimated based on magnetic data and a change in a current waveform in a virtual electrode set in the region-of-interest;
FIG. 9 is a diagram illustrating an example of a method of installing a virtual electrode along a position of a nerve in FIG. 6 or FIG. 8;
FIG. 10 is a configuration diagram illustrating an example of a biomagnetism measurement system including a biomagnetism measurement apparatus according to a second embodiment of the present invention;
FIG. 11 is a flow diagram illustrating an example of an operation of the biomagnetism measurement apparatus of FIG. 10; and
FIG. 12 is a block diagram illustrating an example of a hardware configuration of the biomagnetism measurement apparatus of FIG. 1 and FIG. 10.

### DESCRIPTION OF THE EMBODIMENTS

For example, the biomagnetism measurement apparatus reconstruct current generated in a living body by using an estimation method such as spatial filtering, based on the magnetic data acquired by the sensor array. Then, a neuronal function is evaluated by the current distribution acquired by the reconstruction. At this time, the signal intensity of the magnetic data decreases as the distance between the magnetic source and the sensor increases. In order to reduce the decrease in signal intensity, the position of the nerve to be measured and the position of the sensor are acquired before measuring the biomagnetism, and the acquired position information is provided for the estimation method such as the spatial filtering.

For example, a spinal cord extends in the cranial-caudal direction and the position in the anterior-posterior direction changes, but generally the position in the left-right direction does not change. Therefore, when estimating a nerve activity current of the spinal cord, the reconstructed area of the current specified by the spatial filtering (a virtual electrode) may be set on a surface that bends only in the anterior-posterior direction along the shape of the spinal canal through which the spinal cord passes.

However, for example, a brachial plexus extending outward of the spinal canal extends anteriorly away from the spinal canal toward the shoulder. Therefore, when the reconstructed area in which the current is reconstructed is set in a plane along the shape of the spinal canal, there is a difference between the actual position and the calculated position of the brachial plexus. As a result, accurately estimating the intensity of the nerve activity current of, for example, the brachial plexus extending from the spinal canal becomes difficult.

Embodiments will hereinafter be described with reference to the drawings. In drawings, the same constituent elements are designated by the same reference numerals, and a repeated description of the same constituent elements may be omitted.

### (First Embodiment)

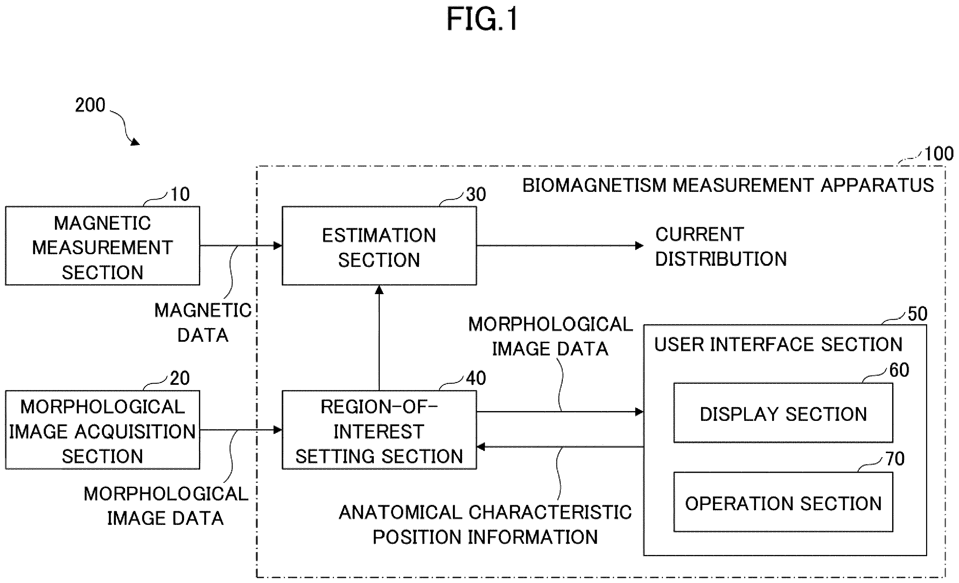

FIG. 1 is a configuration diagram illustrating an example of a biomagnetism measurement system including a biomagnetism measurement apparatus according to a first embodiment of the present invention. For example, a biomagnetism measurement system 200 illustrated in FIG. 1 includes a magnetic measurement section 10, a morphological image acquisition section 20, and a biomagnetism measurement apparatus 100.

The biomagnetism measurement apparatus 100 includes an estimation section 30, a region-of-interest setting section 40, and a user interface section 50. The user interface section 50 includes a display section 60 and an operation section 70. The user interface section 50 may be a tablet or the like. The estimation section 30 and the region-of-interest setting section 40 may be implemented by executing a biomagnetism measurement program by a processor such as a Central Processing Unit (CPU) provided in the biomagnetism measurement apparatus 100.

For example, the magnetic measurement section 10 includes a signal processor and a superconducting quantum interference device (SQUID) sensor array including multiple SQUIDs. The magnetic measurement section 10 measures the magnetic field generated by the electrical activity of the nerve in the portion to be evaluated of the subject (a living body) in response to electrical stimulation by a nerve stimulator or the like. The magnetic measurement section 10 outputs the measured magnetic field as magnetic data to the estimation section 30. In the present embodiment, the magnetic measurement section 10 is used as a magnetospinograph (MSG).

For example, the morphological image acquisition section 20 is included in an X-ray apparatus, a computed tomography (CT) imaging apparatus, or a magnetic resonance imaging apparatus. Hereinafter, the magnetic resonance imaging apparatus is also referred to as an MRI apparatus.

The morphological image acquisition section 20 acquires a morphological image including the portion to be evaluated of the subject. The morphological image acquisition section 20 is an example of an image acquisition section that acquires a morphological image. The morphological image may be an X-ray image, a CT image, or an MRI image. The morphological image acquisition section 20 outputs the acquired morphological image as morphological image data to the region-of-interest setting section 40. Note that the X-ray apparatus and the morphological image acquisition section 20 included in the X-ray apparatus are disposed on the front side and the lateral side of the subject.

The estimation section 30 estimates the current distribution in the region-of-interest set by the region-of-interest setting section 40 based on the magnetic data received from the magnetic measurement section 10 and outputs the estimated current distribution. For example, the current distribution estimated by the estimation section 30 may be displayed on the display section 60 together with the morphological image. The region-of-interest is a computational area of an estimation algorithm used in estimation techniques such as the spatial filtering.

The estimation section 30 may use the estimated current distribution to acquire a current waveform estimated at the virtual electrode set by the operation section 70 based on the operator's operation. The current waveform acquired by the estimation section 30 is displayed on the display section 60. The estimation section 30 is an example of a current waveform acquisition section that acquires the estimated current waveform using a virtual electrode. Note that the current waveform acquisition section may be provided external to the estimation section 30. In this case, the current waveform acquisition section acquires the current waveform at the virtual electrode set by the operation section 70 using the current distribution estimated by the estimation section 30.

The region-of-interest setting section 40 outputs the morphological image data received from the morphological image acquisition section 20 to the user interface section 50. The region-of-interest setting section 40 receives anatomical characteristic position information from the user interface section 50.

When a portion to be evaluated for nerve activity is a nerve around the cervical spine such as the brachial plexus, the anatomical characteristic position information includes, for example, position information medial of the coracoid process and position information of an anterior margin of a seventh cervical vertebra. When the portion to be evaluated for nerve activity is a nerve around the lumbar spine such as a lumbar plexus, the anatomical characteristic position information includes position information of a greater sciatic notch and position information of an intersection of a posterior margin of a first sacral vertebra and an inferior margin of a pedicle.

The region-of-interest setting section 40 resets a predetermined reference region-of-interest based on the anatomical characteristic position information received from the user interface section 50. The region-of-interest setting section 40 outputs information indicating the region-of-interest that has been reset to the estimation section 30. The reference region-of-interest is indicated by a curved surface in which the anterior-posterior position changes in the cranial-caudal direction with respect to the subject. After resetting, the region-of-interest is indicated by a curved surface in which each of the anterior-posterior position in the cranial-caudal direction with respect to the subject and the anterior-posterior position in the left-right direction with respect to the subject changes.

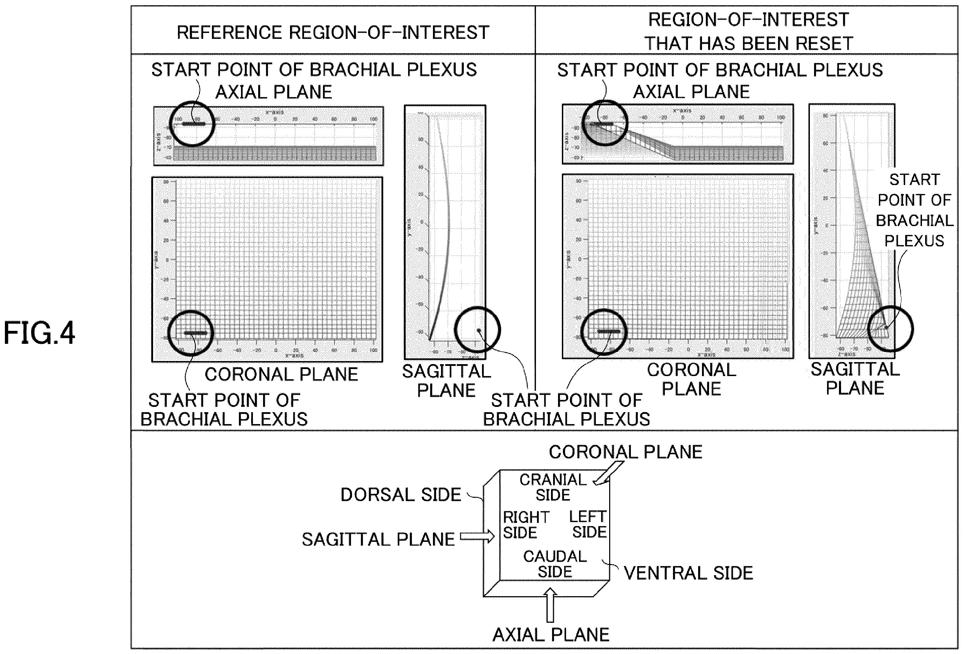

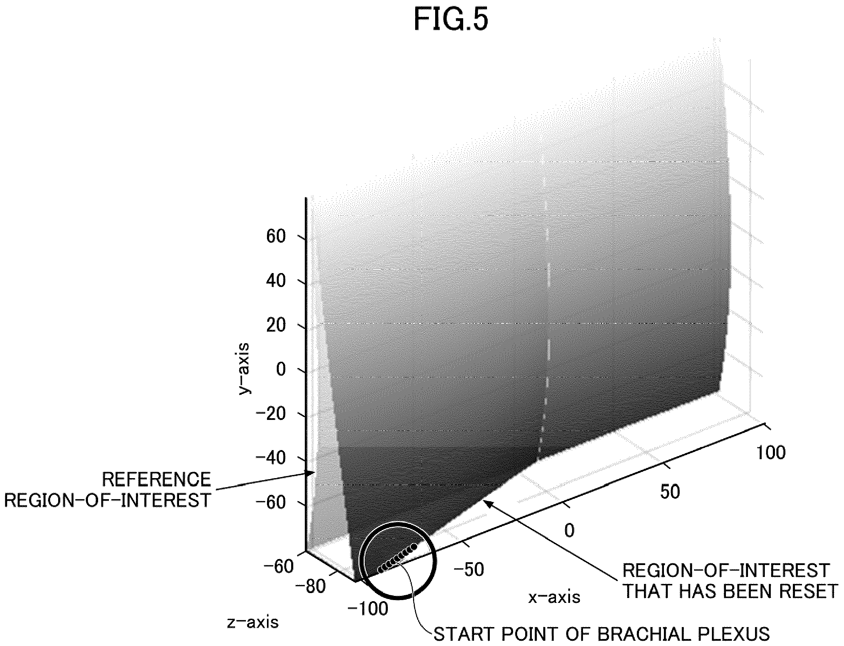

When the biomagnetism measurement apparatus 100 estimates the nerve activity current of nerves of the cervical spinal cord and nerves around the cervical spine (for example, brachial plexus), the region-of-interest setting section 40 resets the region-of-interest to a curve including a position 2 cm to 4 cm from the medial of the coracoid process to the center side and a depth position of the center of the anterior margin of the seventh cervical vertebra. Note that the nerve activity current of which the nerve is estimated by the biomagnetism measurement apparatus 100 is specified in advance by an operator through the operation section 70. Examples of the reference region-of-interest and the region-of-interest that has been reset for estimating the nerve activity current of the cervical spinal cord and brachial plexus are illustrated in FIG. 4 and FIG. 5.

When the biomagnetism measurement apparatus 100 estimates the nerve activity current of nerves of the lumbar cord and nerves around the lumbar spine (for example, lumbar plexus), the region-of-interest setting section 40 resets the region-of-interest to a curve including the position of the greater sciatic notch and the depth position of the intersection of the posterior margin of the first sacral vertebra and the inferior margin of the pedicle.

The display section 60 has a function of displaying the morphological image acquired by the morphological image acquisition section 20 on a screen. The display section 60 has a function of displaying the current distribution estimated by the estimation section 30 on the screen.

The operation section 70 receives an anatomical characteristic position that is an indicator of the position of the nerve to be evaluated, which is input based on the morphological image displayed on the screen of the display section 60. For example, an operator viewing the morphological image displayed on the screen of the display section 60 selects the anatomical characteristic position on the morphological image displayed on the screen. For example, when evaluating the nerve activity of the brachial plexus, the operator may use a mouse or the like connected to the user interface section 50 to specify the position of the medial of the coracoid process and the anterior margin of the seventh cervical vertebra as seen in the morphological image.

The operation section 70 detects a coordinate of the anatomical characteristic position selected by the operator on the screen and outputs the detected coordinate to region-of-interest setting section 40 as the anatomical characteristic position information. The region-of-interest setting section 40 resets the region-of-interest based on the coordinate information of the morphological image indicated by the morphological image data and the anatomical characteristic position information (the coordinate, etc.) received from the operation section 70.

Further, the operation section 70 sets a virtual electrode, based on the operation by the operator, on the position of the nerve to be evaluated positioned in the region-of-interest represented by a three-dimensional space, which is displayed on the display section 60 together with the morphological image. The method of representing the region-of-interest on the three-dimensional space will be described later. The operation section 70 is an example of a virtual electrode setting section that sets a virtual electrode on the position of the nerve to be evaluated along the region-of-interest represented in three dimensions.

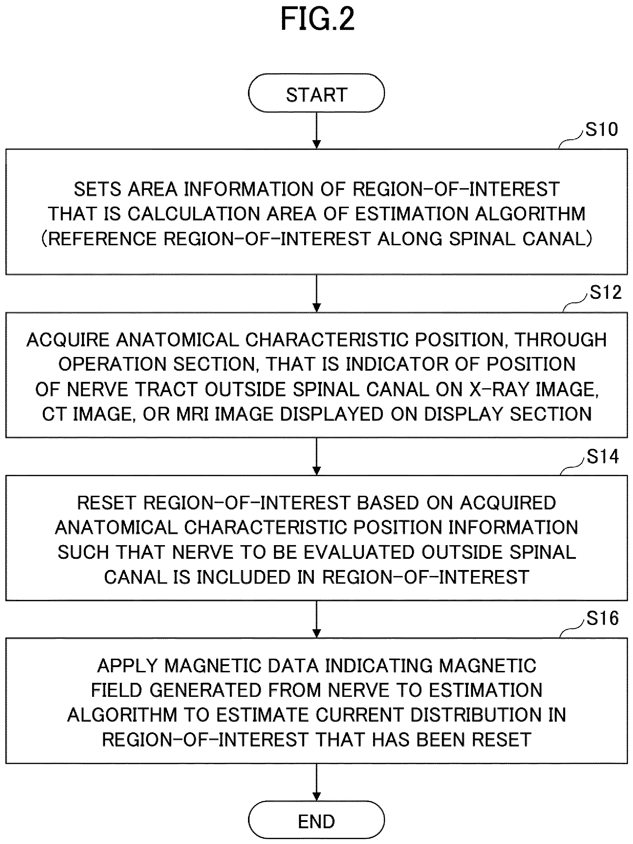

FIG. 2 is a flow diagram illustrating an example of an operation of the biomagnetism measurement apparatus 100 of FIG. 1. For example, the flow illustrated in FIG. 2 is implemented by executing a biomagnetism measurement program by a processor such as a CPU mounted in the biomagnetism measurement apparatus 100. That is, FIG. 2 illustrates an example of a biomagnetism measurement method and a biomagnetism measurement program executed by the biomagnetism measurement apparatus 100.

First, in step S10, the region-of-interest setting section 40 sets the area information of the region-of-interest that is the calculation area of the estimation algorithm. The region-of-interest set by step S10 is the reference region-of-interest, including a surface along the spinal canal, and is indicated by a curved surface in which the anterior-posterior position changes in the cranial-caudal direction with respect to the subject, as described above.

Next, in step S12, the region-of-interest setting section 40 acquires the anatomical characteristic position information through the operation section 70. The anatomical characteristic position information indicates the anatomical characteristic position that is an indicator of the position of the nerve to be evaluated on the X-ray image, the CT image, or the MRI image displayed on the display section 60. For example, the operation section 70 receives the anatomical characteristic position information based on the operation by the operator who views the morphological image. Note that step S10 and step S12 may be performed in the reverse order.

Occasionally, nerves and muscles are not visible in the X-ray image and the CT images, and are difficult to be visible in the MRI image. Meanwhile, a position (anatomical characteristic position) of the bones, such as the coracoid process, the seventh cervical vertebra, and the greater sciatic notch, can be determined from the X-ray image, the CT image, and the MRI image. Accordingly, the region-of-interest setting section 40 can determine the position of the nerve, such as the brachial plexus, the lumbar plexus, or the like, which is not visible or hardly visible in the image, based on the anatomical characteristic position information received from the operation section 70.

Next, in step S14, the region-of-interest setting section 40 resets the region-of-interest based on the acquired anatomical characteristic position information such that the nerve to be evaluated outside the spinal canal is included in the region-of-interest. This enables the region-of-interest setting section 40 to set the region-of-interest to a curved surface in which the anterior-posterior position in the cranial-caudal direction with respect to the subject and the anterior-posterior position in the left-right direction with respect to the subject change. As a result, the region-of-interest can be set on a curved surface including the brachial plexus or the lumbar plexus as well as the spinal cord.

Next, in step S16, the estimation section 30 applies magnetic data indicating a magnetic field generated from the nerve to be evaluated to the estimation algorithm to estimate the current distribution in the region-of-interest that has been reset. In this case, the curved surface indicating the region-of-interest includes not only the position of the spinal cord but also the position of the brachial plexus or the lumbar plexus.

Therefore, the estimation section 30 can calculate the nerve activity current at the actual position of the brachial plexus or the lumbar plexus, which is another portion to be evaluated, as well as the nerve activity current of the cervical spinal cord or the lumbar cord, which is one of the portion to be evaluated. This enables correct estimation of the nerve activity current of the brachial plexus or the lumbar plexus, which is being apart from the spinal canal in the anterior-posterior direction with respect to the subject, together with the nerve activity current of the spinal cord.

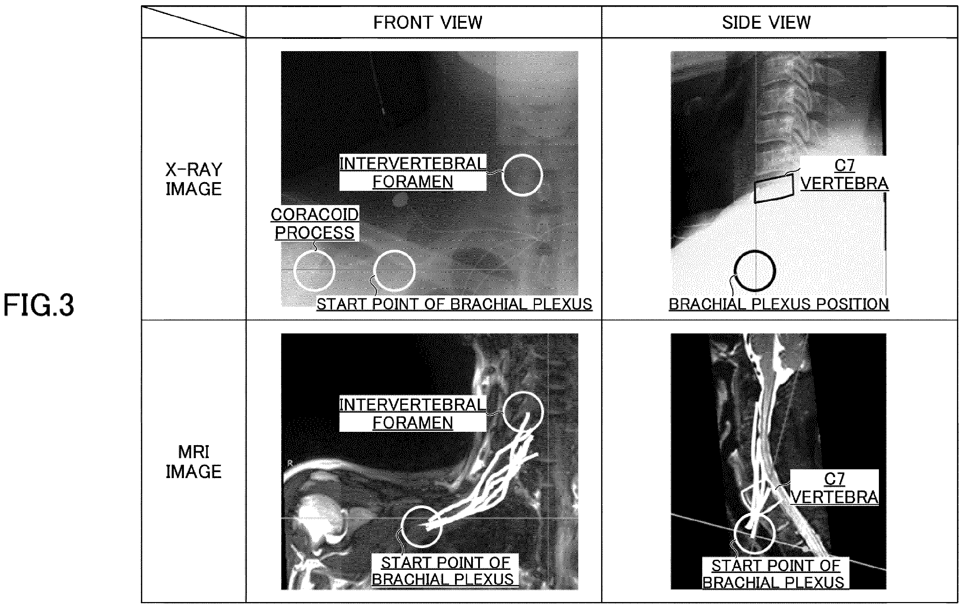

FIG. 3 is a diagram illustrating an example of the morphological image of the subject acquired by the morphological image acquisition section 20 of FIG. 1. The morphological images illustrated in FIG. 3 illustrate both the cervical spine and around the cervical spine. The morphological image on the left side of FIG. 3 illustrates the subject taken from the front. The morphological image on the right side of FIG. 3 illustrates the subject taken from the right side.

The upper side of FIG. 3 illustrates an example of an X-ray image acquired by the morphological image acquisition section 20 included in the X-ray apparatus. The lower side of FIG. 3 illustrates an example of an MRI image acquired by the morphological image acquisition section 20 included in the MRI apparatus. The operator who operates the operation section 70 (FIG. 1) observes the X-ray image or the MRI image of the front view and the side view displayed on the display section 60 (FIG. 1), and when the brachial plexus is included in the nerve to be measured, the medial of the coracoid process and the anterior margin of the seventh cervical vertebra are specified on the screen. The position on the screen specified by the operator is output to the region-of-interest setting section 40 as the anatomical characteristic position information.

When the morphological image acquisition section 20 is included in a CT imaging apparatus, the operator observes the CT image by sequentially displaying the cross-sectional image of the subject on the display section 60 while changing the cross-sectional position, and specifies the medial of the coracoid process and the anterior margin of the seventh cervical vertebra on the screen.

Based on the anatomical characteristic position information received from the user interface section 50, when the brachial plexus is included in the nerve to be measured, the region-of-interest setting section 40 includes a position in the range of 2 cm to 4 cm from the position of the medial of the coracoid process (on the spinal canal side) to the center side and a position of the center in the height direction of the anterior margin of the seventh cervical vertebra into the region-of-interest. In this regard, the position 2 cm to 4 cm from the medial of the coracoid process to the spinal cord side is a start point of the brachial plexus in the left-right direction, with individual differences between the subjects being taken into consideration. Further, the center in the height direction of the anterior margin of the seventh cervical vertebra is a start point of the anterior-posterior direction of the brachial plexus.

The region-of-interest setting section 40 also includes the position of the intervertebral foramen, which is an end point of the brachial plexus, in the region-of-interest. Further, the region-of-interest setting section 40 includes a straight line connecting the end point from the start point of the brachial plexus in the region-of-interest.

Note that the region-of-interest setting section 40 may set a line connecting the coracoid process side to the depth position to be a curved line instead of the straight line, as long as the line is included in the range indicating the position of the brachial plexus. Further, when acquiring the anatomical characteristic position from the CT image or the MRI image, a variation of the position of an upper vertebral body due to the posture is greater than a variation of the position of a lower vertebral body due to the posture. Therefore, the vertebral body, which is the reference anatomical characteristic position, on the lower side (for example, the seventh cervical vertebra, which is in the lowest position) is preferable.

The region-of-interest setting section 40 resets the region-of-interest by transforming a flat surface having a bent shape indicating the reference region-of-interest into the curved surface including the brachial plexus. This enables setting of set a curved surface including both a nerve route of the spinal cord and a nerve route of the brachial plexus as the region-of-interest. The multiple white lines connecting the start point of the brachial plexus and the intervertebral foramen indicated in the MRI image illustrated in FIG. 3 are added for imaging the brachial plexus. Note that the white lines are not included in the actual MRI image.

FIG. 4 is a diagram illustrating an example of the region-of-interest set by the region-of-interest setting section 40 in FIG. 1. The left side of FIG. 4 illustrates an example of a reference region-of-interest set by step S10 of FIG. 2. The right side of FIG. 4 illustrates an example of the region-of-interest that has been reset by step S14 of FIG. 2. In FIG. 4, the curved surface indicating the region-of-interest is illustrated in mesh form in the coronal plane view, the sagittal plane view, and the axial plane view.

The coronal plane view indicates that the subject is viewed from the front. Therefore, the left side of the figure corresponds to the right side of the subject and the right side of the figure corresponds to the left side of the subject. Further, the upper side of the figure corresponds to the cranial side (upper side) of the subject and the lower side corresponds to the caudal side (lower side) of the subject.

The sagittal plane view illustrates the subject viewed from the right side. Therefore, the left side of the figure corresponds to the dorsal (posterior) side of the subject, and the right side corresponds to the ventral (anterior) side of the subject. Further, the upper side of the figure corresponds to the cranial side of the subject and the lower side corresponds to the caudal side of the subject.

The axial plane view illustrates a supine subject viewed from the caudal side. Therefore, the left side of the figure corresponds to the right side of the subject and the right side of the figure corresponds to the left side of the subject. Further, the upper side of the figure corresponds to the ventral side of the subject, and the lower side corresponds to the dorsal side of the subject.

The black dots in the circles of the figure indicate the start point of the brachial plexus (2 to 4 cm range) estimated from the coracoid process, which is the anatomical characteristic position indicated by the morphological image. The brachial plexus extends from the lower side of the medial of the coracoid process and the anterior margin of the seventh cervical vertebra toward the intervertebral foramen in the direction of the spinal canal, as illustrated in FIG. 3.

Therefore, the position of the brachial plexus is not included in the mesh indicating the reference region-of-interest set to the bent shape along the spinal canal. The reference region-of-interest is indicated by a straight line with a uniform cross-sectional shape in the left-right direction as illustrated on the axial plane, and is indicated by a curve with a uniform cross-sectional shape in the cranial-caudal direction as illustrated on the sagittal plane.

Meanwhile, since the mesh of the region-of-interest that has been reset is set to a curved shape along the spinal canal and the brachial plexus, the position of the brachial plexus is included in the mesh indicating the region-of-interest that has been reset. The region-of-interest that has been reset does not have a cross-sectional shape with a straight line in the left-right direction as illustrated on the axial plane, and the cross-sectional shape in the cranial-caudal direction is not a uniform curve as illustrated on the sagittal plane. The mesh of the region-of-interest that has been reset can be represented by a quadratic curved surface that is a curved surface whose cut end by a plane is a quadric curve such as an ellipse, a hyperbola, a parabola, or the like. The quadratic surface is represented by a three-dimensional quadratic equation.

FIG. 5 is a perspective view illustrating the reference region-of-interest of FIG. 4 and the region-of-interest that has been reset. As illustrated in FIG. 5, the reference region-of-interest is set to a curved surface shape along the curved shape of the spinal canal. In contrast, the region-of-interest that has been reset has a shape along the curved shape of the spinal canal in the portion corresponding to the spinal canal, and a shape along the tract of the brachial plexus.

The estimation section 30 illustrated in FIG. 1 reconstructs the current generated in the living body based on the magnetic data by using the estimation method such as the spatial filtering, assuming that a virtual electrode is set on the region-of-interest that has been reset. Therefore, the nerve activity current of the brachial plexus positioned on the region-of-interest can be correctly estimated together with the nerve activity current of the spinal cord.

Conventionally, measurements of the brachial plexus were difficult to evaluate in the supine position, where the subject was in a less burdensome position. The brachial plexus changes greatly in the depth direction, which is not visible in the X-ray image, so estimating the intensity accurately was difficult. Therefore, conventional measurements were performed in the prone position.

In the prone position, pushing the brachial plexus against the sensor section reduces the change in the depth direction of the nerve route. Therefore, relatively accurate intensity estimation was possible. On the other hand, in the prone position, detecting a biomagnetism field signal of the cervical spinal cord was difficult because the distance between the cervical spinal cord and the sensor was long. Even if the cervical spinal cord was detected, the nerve activity during rest cannot be asserted to be correctly reflected because the neck was twisted, and thus evaluating was difficult.

Therefore, evaluating a nerve function consistently from the brachial plexus to the cervical spinal cord was difficult. Further, from the viewpoint of the burden on the subject, a method to enable measurement in the supine position, which is generally a position with less burden, was desired to be developed. In the present embodiment, the brachial plexus can be measured in the supine position by inputting an anatomical positional relationship of the brachial plexus and setting the region-of-interest along the brachial plexus. This enables the brachial plexus and the neck to be measured without changing position in a series of flows.

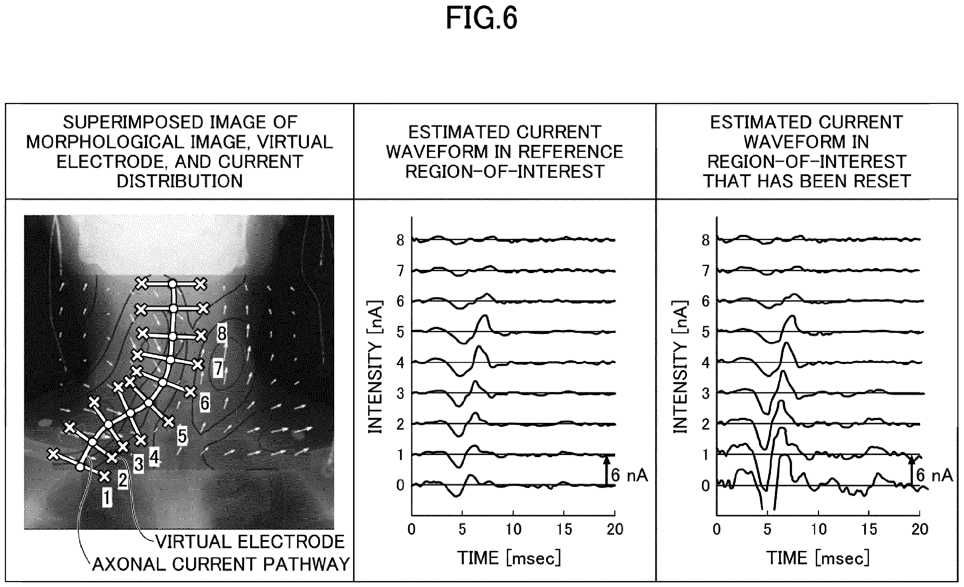

FIG. 6 is a diagram illustrating an example of the current distribution in the region-of-interest estimated based on the magnetic data and a change in the current waveform in the virtual electrode set in the region-of-interest. FIG. 6 illustrates an estimation result of the current distribution when electrical stimulation is applied to the right hand of the subject.

The left side of FIG. 6 illustrates an image in which the morphological image (X-ray image) of the neck of the subject, the virtual electrode, an intra-axonal current pathway, and the current distribution in the region-of-interest setting section 40 are superimposed. The image on the left side of FIG. 6 corresponds to the coronal plane view of FIG. 4. Setting of the virtual electrode on the morphological image of the subject can be performed by the user specifying a position on the morphological image displayed on the display section 60 using a mouse or the like connected to the user interface section 50. In this case, the virtual electrode can be automatically set at a predetermined interval.

The middle and right sides of FIG. 6 illustrate the estimated current waveform in the reference region-of-interest and the estimated current waveform in the region-of-interest that has been reset, respectively. The number indicated on the intensity axis of the current waveform corresponds to the number of the virtual electrode in the morphological image. When the electrical stimulation is applied from the right hand, the waveform of the current flowing through the virtual electrode indicated by the X mark positioned on the left side, which is customarily opposite, is evaluated.

From the two current waveforms, the current according to the electrical stimulation propagates sequentially from the virtual electrode having a small number to the virtual electrode having a large number. However, in the current waveform on the left side, a virtual electrode whose signal intensity increases with propagation is exists, and the estimated result cannot be explained neurophysiologically.

In the current waveform on the right side, the intensity of the nerve activity current flowing through the brachial plexus is greater than the intensity of the current waveform on the left side. Further, in the current waveform on the right side, the intensity of the nerve activity current decreases with propagation, and the estimated result is acceptable neurophysiologically. As described above, the nerve activity current cannot be correctly estimated in the reference region-of-interest, whereas the nerve activity current can be correctly estimated in the region-of-interest that has been reset. The same applies to the case of the lumbar plexus.

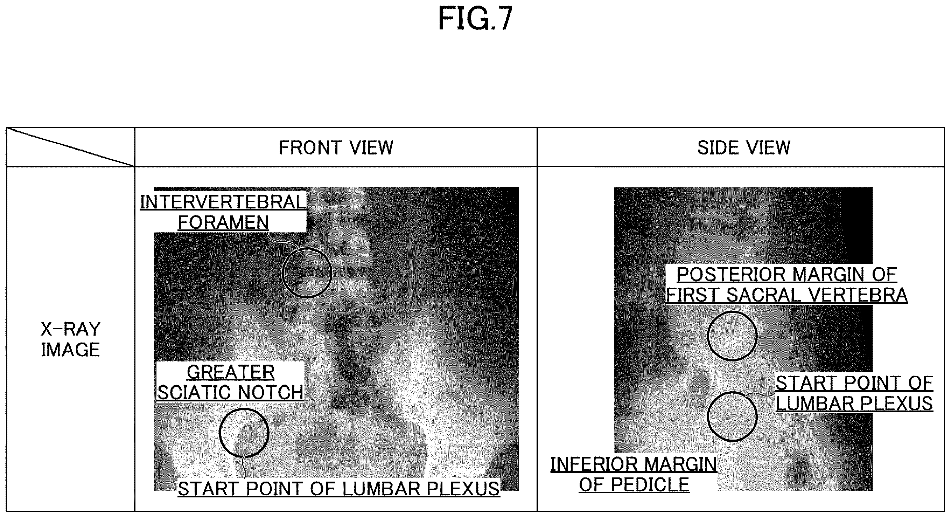

FIG. 7 is a diagram illustrating another example of a morphological image of the subject acquired by the morphological image acquisition section of FIG. 1. The morphology image of FIG. 7 illustrates the lumbar spine and around the lumbar spine. The morphological image on the left side of FIG. 7 illustrates the subject taken from the front. The morphological image on the right side of FIG. 7 illustrates the subject taken from the right side. FIG. 7 illustrates an example of an X-ray image acquired by the morphological image acquisition section 20 included in the X-ray apparatus.

The operator who operates the operation section 70 (FIG. 1) observes the X-ray image of the front view and the side view displayed on the display section 60 (FIG. 1), and when the lumbar plexus is included in the nerve to be measured, the operator specifies the anatomical characteristic position which is an indicator of the position of the lumbar plexus on the screen. The position on the screen specified by the operator is output to the region-of-interest setting section 40 as the anatomical characteristic position information.

The anatomical characteristic position that is an indicator of the position of the lumbar plexus is, for example, the position of the greater sciatic notch and the depth position of the intersection of the posterior margin of the first sacral vertebra and the inferior margin of the pedicle. In this regard, the position of the greater sciatic notch is the start point of the left-right direction of the lumbar plexus, and the depth position of the intersection of the posterior margin of the first sacral vertebra and the inferior margin of the pedicle is the start point of the anterior-posterior direction of the lumbar plexus. Similar to the brachial plexus, the end point of the lumbar plexus is the intervertebral foramen. This enables the nerves of the lumbar cord and the nerves around the lumbar spine to be evaluated for the nerve activity current.

When the morphological image acquisition section 20 is included in a CT imaging apparatus, the operator observes the CT image by sequentially displaying the cross-sectional image of the subject on the display section 60 while changing the cross-sectional position, and specifies the position of the greater sciatic notch and the depth position of the intersection of the posterior margin of the first sacral vertebra and the inferior margin of the pedicle.

Based on the anatomical characteristic position information received from the user interface section 50, when the lumbar plexus is included in the nerve to be measured, the region-of-interest setting section 40 includes the position of the greater sciatic notch and the depth position of the intersection of the posterior margin of the first sacral vertebra and the inferior margin of the pedicle.

Further, the region-of-interest setting section 40 includes a straight line connecting the position of the greater sciatic notch and the depth position of the intersection of the posterior margin of the first sacral vertebra and the inferior margin of the pedicle. Note that the region-of-interest setting section 40 may set a line connecting the position of the greater sciatic notch and the depth position of the intersection of the posterior margin of the first sacral vertebra and the inferior margin of the pedicle to be a curved shape instead of the straight line, as long as the line is included in the range indicating the position of the brachial plexus.

The region-of-interest setting section 40 resets the region-of-interest by transforming a surface having a bent shape indicating the reference region-of-interest into the curved surface including the lumbar plexus, similar to the reset of the region-of-interest including the brachial plexus illustrated in FIG. 4 and FIG. 5. This enables setting of a curved surface including both a nerve route of the spinal cord and a nerve route of the lumbar plexus as the region-of-interest.

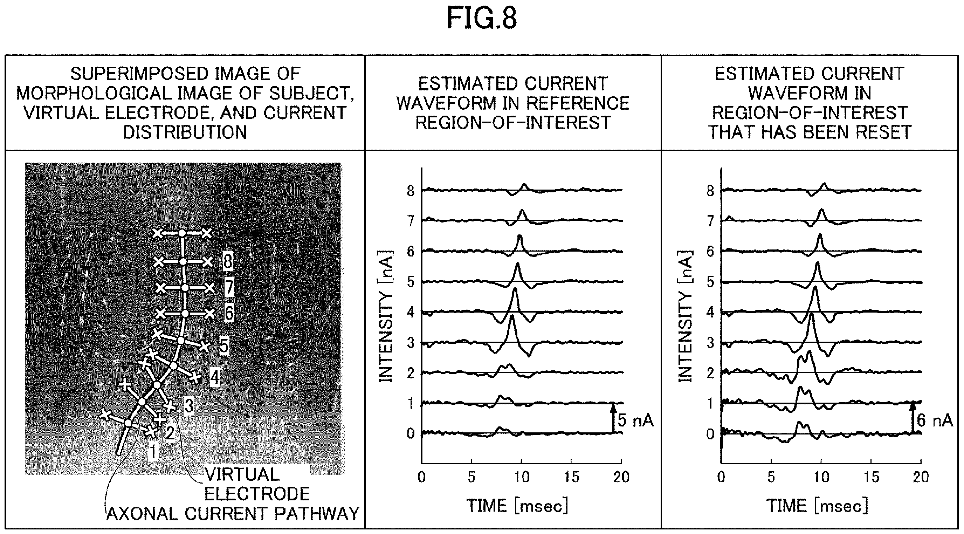

FIG. 8 is a diagram illustrating another example of a current distribution in the region-of-interest estimated based on the magnetic data and a change in the current waveform in the virtual electrode set in the region-of-interest. Detailed description of the same elements as in FIG. 6 will be omitted. FIG. 8 illustrates an estimation result of the current distribution when the right foot of the subject is electrically stimulated.

The left side of FIG. 8 illustrates an image in which the morphological image (X-ray image) of the lumbar of the subject, the virtual electrode, the intra-axonal current pathway, and the current distribution in the region-of-interest setting section 40 are superimposed. The middle and the right side of FIG. 8 illustrate the estimated current waveform in the reference region-of-interest and the estimated current waveform in the region-of-interest that has been reset, respectively. Similar to FIG. 6, a setting of the virtual electrode on the morphological image of the subject can be performed by the user specifying a position on the morphological image displayed on the display section 60 using a mouse or the like connected to the user interface section 50. In this case, the virtual electrode can be automatically set at a predetermined interval.

From the two current waveforms, the current according to the electrical stimulation propagates sequentially from the virtual electrode having a small number to the virtual electrode having a large number. However, in the current waveform on the left side, similarly to the current waveform on the left side in FIG. 6, a virtual electrode whose signal intensity increases with propagation is exists, and the estimated result cannot be explained neurophysiologically. In the current waveform on the right side, similarly to the current waveform on the right side in FIG. 6, the intensity of the nerve activity current flowing through the lumbar plexus is greater than the intensity of the current waveform on the left side. Further, in the current waveform on the right side, the intensity of the nerve activity current decreases with propagation, and the estimated result is acceptable neurophysiologically. As described above, also in the lumbar plexus, the nerve activity current cannot be correctly estimated in the reference region-of-interest, whereas the nerve activity current can be correctly estimated in the region-of-interest that has been reset.

As described above, in the present embodiment, the biomagnetism measurement apparatus 100 can determine the position of the nerve, such as the brachial plexus, the lumbar plexus, or the like, which is not visible or is difficult to make out, by acquiring the anatomical characteristic position information through the operation section 70. Then, the region-of-interest setting section 40 can set, based on the determined position of the nerve, the region-of-interest including the position of the nerve, such as the brachial plexus, the lumbar plexus, or the like, which is apart from the spinal cord. As a result, the biomagnetism measurement apparatus 100 can correctly estimate the nerve activity current outside the spinal canal together with the nerve activity current of the spinal cord.

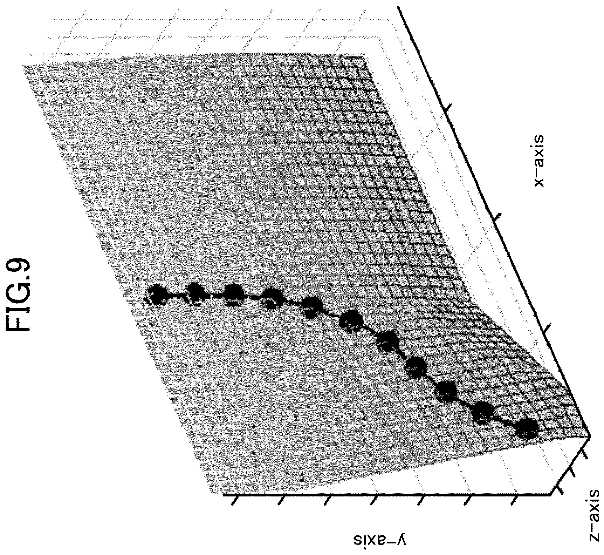

Next, a method of setting a virtual electrode along the position of the nerve in the three-dimensional space will be described. FIG. 9 is a diagram illustrating an example of a method of setting the virtual electrode along the position of the nerve in FIG. 6 or FIG. 8. In FIG. 9, an area indicated by the mesh illustrates, for example, the region-of-interest that has been reset illustrated in FIG. 5. The black line indicates the position of the nerve (the intra-axonal current pathway), and the black dot indicates the position of the virtual electrode. The region-of-interest and each axis illustrated in FIG. 9 are illustrated in the display section 60, for example, as a perspective view.

For example, after superimposing the intra-axonal current pathway and the virtual electrode (superimposed image on the left side of FIG. 6 or FIG. 8) on the morphological image (X-ray image, etc.) of the subject on the two-dimensional plane by the user interface section 50, the virtual electrode in the three-dimensional space can be set by resetting the intra-axonal current pathway on the two-dimensional plane and the virtual electrode in the three-dimensional space along the region-of-interest of FIG. 9.

For example, superimposing the intra-axonal current pathway and the virtual electrode on the morphological image (X-ray image, etc.) of the subject on the two-dimensional plane can be implemented by the user specifying a position on the morphological image displayed on the display section 60 using a mouse or the like connected to the user interface section 50. In this case, the virtual electrode can be automatically set at a predetermined interval.

Further, the virtual electrode in the three-dimensional space may be directly set on the region-of-interest of FIG. 9 by the user interface section 50. In this case, for example, first, the morphological image of the subject and the region-of-interest of FIG. 9 corresponding to the morphological image are displayed on the display section 60. Next, a mouse or the like connected to the user interface section 50 is used by the user to set at least one of the intra-axonal current pathway and the virtual electrode on the region-of-interest displayed by the mesh on the display section 60. When the user sets only the virtual electrode on the region-of-interest, the user interface section 50 may automatically set a path passing through the virtual electrode arranged on the region-of-interest as the intra-axonal current pathway. When the user sets only the intra-axonal current pathway on the region-of-interest, the user interface section 50 may automatically set the virtual electrode on the region-of-interest at a predetermined interval.

Note that a display angle of the region-of-interest illustrated in FIG. 9 is set to a suitable angle to facilitate the user to set the intra-axonal current pathway or the virtual electrode on the region-of-interest. However, the display angle of the region-of-interest may be adjustable based on an instruction from the user. This allows the user to finely adjust the position of the intra-axonal current pathway or the virtual electrode in the region-of-interest while changing the display angle, and to precisely set the position of the intra-axonal current pathway and the virtual electrode. In this regard, if the display section 60 is capable of stereoscopically displaying the morphological image, such as the MRI image or the CT image, the user interface section 50 may adjust the display angle of the morphological image according to the adjustment of the display angle of the region-of-interest.

Therefore, the virtual electrode can be set in three dimensions according to the actual position of the nerve in the three-dimensional space including z-axis, rather than a plane represented by x-axis and y-axis. Then, by acquiring the current waveform estimated by the set virtual electrode, the user can evaluate the nerve function by correctly reflecting the position of the nerve route. Further, the user can reset the region-of-interest via the user interface section 50 after setting the position of the nerve. After resetting, the user can re-estimate the nerve activity current to acquire the current waveform of the position of the virtual electrode. When the region-of-interest is reset, the intra-axonal current pathway and the virtual electrode on the region-of-interest are also reset.

### (Second Embodiment)

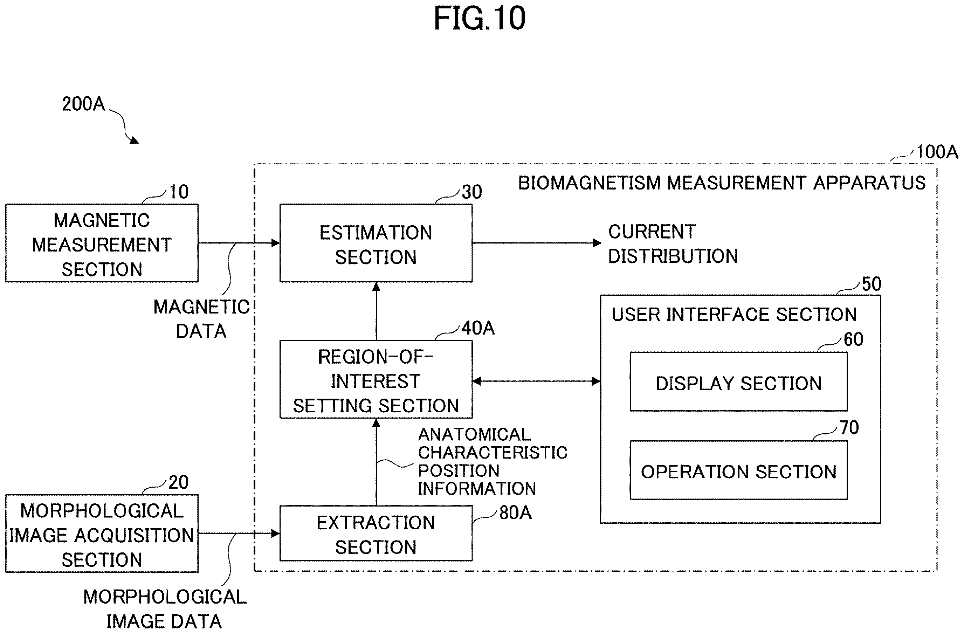

FIG. 10 is a configuration diagram illustrating an example of a biomagnetism measurement system including a biomagnetism measurement apparatus according to a second embodiment of the present invention. The same components as those in FIG. 1 are designated by the same reference numerals, and detailed description thereof will be omitted. For example, a biomagnetism measurement system 200A illustrated in FIG. 10 includes a magnetic measurement section 10, a morphological image acquisition section 20, and a biomagnetism measurement apparatus 100A.

The biomagnetism measurement apparatus 100A includes an estimation section 30, a region-of-interest setting section 40A, an extraction section 80A, and a user interface section 50. The estimation section 30, the region-of-interest setting section 40A, and the extraction section 80A may be implemented by executing a biomagnetism measurement program by a processor such as a CPU provided in the biomagnetism measurement apparatus 100A.

The extraction section 80A uses morphological image data received from the morphological image acquisition section 20 to extract an anatomical characteristic position (medial of the coracoid process and position information of the anterior margin of the seventh cervical vertebra, or the like) which is an indicator of the position of a nerve to be evaluated. Then, the extraction section 80A outputs anatomical characteristic position information indicating the extracted anatomical characteristic position to the region-of-interest setting section 40A. That is, in the present embodiment, the biomagnetism measurement apparatus 100A can automatically extract the anatomical characteristic position that is an indicator of the position of the nerve to be evaluated without receiving an instruction from an operator.

The region-of-interest setting section 40A has a function similar to that of the region-of-interest setting section 40 of FIG. 1, except that the region-of-interest setting section 40A receives the anatomical characteristic position information from the extraction section 80A instead of receiving the anatomical characteristic position information from the user interface section 50. Then, the region-of-interest setting section 40A transforms the curved surface indicating the reference region-of-interest based on the anatomical characteristic position extracted by the extraction section 80A to reset the region-of-interest.

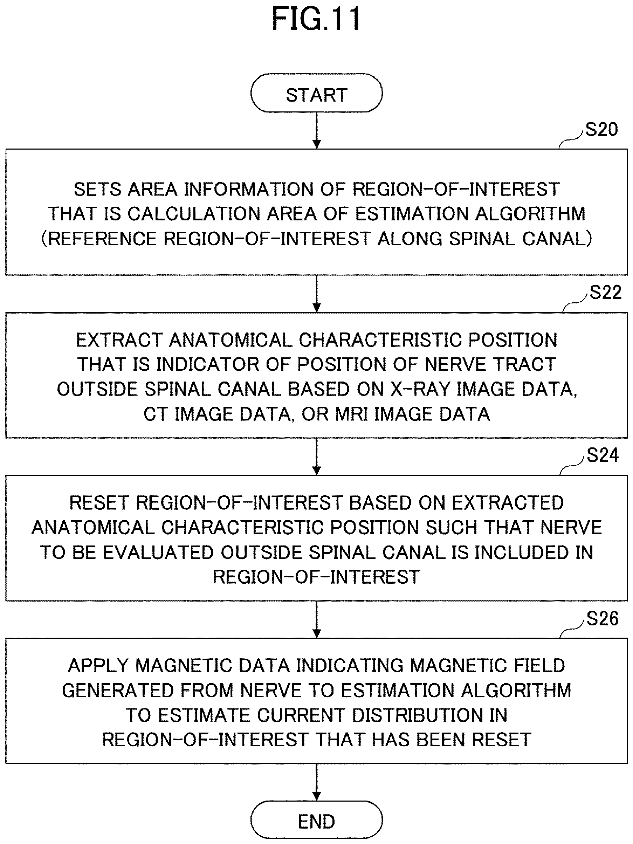

FIG. 11 is a flow diagram illustrating an example of an operation of the biomagnetism measurement apparatus illustrated in FIG. 10. A detailed description of the same operation as in FIG. 2 will be omitted. For example, a flow illustrated in FIG. 11 is implemented by executing a biomagnetism measurement program by a processor such as a CPU provided in the biomagnetism measurement apparatus 100A. That is, FIG. 11 illustrates an example of a biomagnetism measurement method and a biomagnetism measurement program executed by the biomagnetism measurement apparatus 100A.

In step S20 and step S26, the same processing as in step S10 and step S16 of FIG. 2 is performed. After step S20, in step S22, the extraction section 80A extracts the anatomical characteristic position that is an indicator of the position of the nerve route outside the spinal canal based on the X-ray image data, the CT image data, or the MRI image data. For example, the extraction section 80A extracts as the anatomical characteristic position, such as the medial of the coracoid process and the position information of the anterior margin of the seventh cervical vertebra. Note that step S20 and step S22 may be performed in reverse order.

Next, in step S24, the region-of-interest setting section 40A resets the region-of-interest based on the anatomical characteristic position extracted by the extraction section 80A such that the nerve to be evaluated outside the spinal canal is included in the region-of-interest. In step S26, the estimation section 30 estimates the current distribution in the region-of-interest that has been reset based on the magnetic data.

As described above, the same effect as that of the above-described embodiment can be obtained in this embodiment as well. For example, the biomagnetism measurement apparatus 100 may set the region-of-interest including the position of the nerve, such as the brachial plexus, the lumbar plexus, or the like, which is apart from the spinal cord, based on the position of the nerve, such as the brachial plexus, the lumbar plexus, or the like, as determined based on the anatomical characteristic position information. As a result, the biomagnetism measurement apparatus 100 can correctly estimate the nerve activity current outside the spinal canal together with nerve activity current of the spinal cord.

Further, in the present embodiment, the biomagnetism measurement apparatus 100A can automatically extract the anatomical characteristic position that is an indicator of the position of the nerve to be evaluated without receiving an instruction from the operator. Then, the biomagnetism measurement apparatus 100A can reset the region-of-interest including the nerve to be evaluated by determining the position of the nerve, such as the brachial plexus, the lumbar plexus, or the like, based on the automatically extracted anatomical characteristic position.

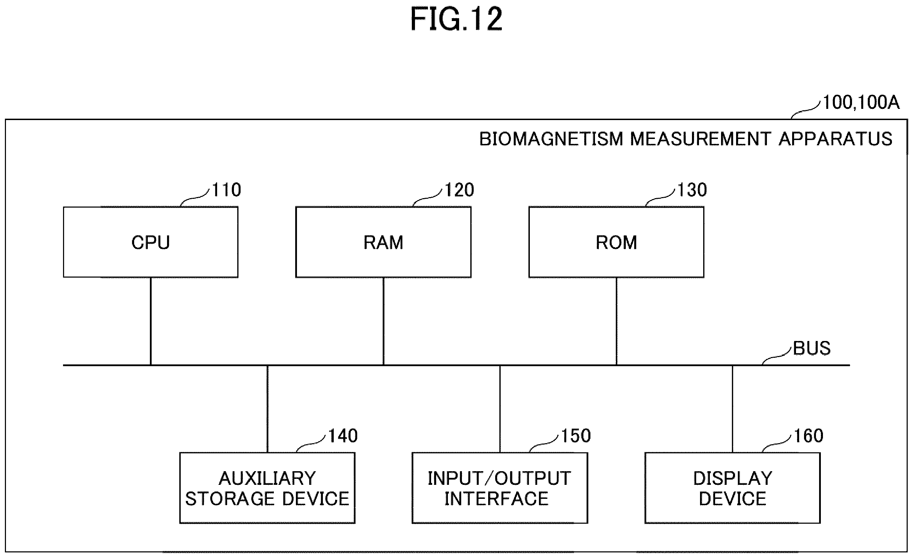

FIG. 12 is a block diagram illustrating an example of a hardware configuration of the biomagnetism measurement apparatus 100 and 100A of FIG. 1 and FIG. 11. Since the hardware configurations of the biomagnetism measurement apparatus 100 and 100A are the same as each other, the configuration of the biomagnetism measurement apparatus 100 will be described below.

For example, the biomagnetism measurement apparatus 100 includes a Central Processing Unit (CPU) 110, a Random Access Memory (RAM) 120, a Read Only Memory (ROM) 130, an auxiliary storage device 140, an input/output interface 150, and a display device 160, each of which is interconnected by a bus BUS. The input/output interface 150 corresponds to the operation section 70. The display device 160 corresponds to the display section 60.

The CPU 110 is an example of a computer. The CPU 110 controls the overall operation of the biomagnetism measurement apparatus 100. The CPU 110 executes the biomagnetism measurement program stored in the ROM 130 or the auxiliary storage device 140 to perform the functions of the estimation section 30 and the region-of-interest setting section 40. In the biomagnetism measurement apparatus 100A, the CPU 110 performs the functions of the estimation section 30, the region-of-interest setting section 40A, and the extraction section 80A by executing the biomagnetism measuring program.

The RAM 120 is used as a work area of the CPU 110 and stores the biomagnetism measuring program and various parameters. The ROM 130 stores the biomagnetism measurement program.

The auxiliary storage device 140 may be a Solid State Drive (SSD) or a Hard Disk Drive (HDD). For example, the auxiliary storage device 140 stores a control program, such as an operating system (OS) for controlling the operation of the biomagnetism measurement apparatus 100, various morphological image, various parameters, and the like.

The input/output interface 150 is connected to a mouse, a keyboard, or the like. The input/output interface 150 may include a communication interface for communicating with other devices. The display device 160 includes, for example, a window for displaying the morphological image and the current waveform illustrated in FIG. 6 and a screen for displaying an operation window.

Although the present disclosure is described heretofore based on the embodiments, the present disclosure is not limited to the described embodiments, and various variations, modifications, and substitutions may be made without departing from the scope of the present disclosure.

The present application claims priority under Japanese Patent Application No. 2021-047934, filed on March 22, 2021 and Japanese Patent Application No. 2021-196264, filed on December 2, 2021, and the entire contents of which are hereby incorporated by reference.

### REFERENCE SIGNS LIST

10 magnetic measurement section
20 morphological image acquisition section
30 estimation section
40, 40A region-of-interest setting section
50 user interface section
60 display section
70 operation section
80A extraction section
100, 100A biomagnetism measurement apparatus
140 auxiliary storage
150 input/output interface
160 display device
200,200A biomagnetism measurement system
BUS bus

## Claims

1. A biomagnetism measurement apparatus comprising:
a region-of-interest setting section configured to set a curved surface as a region-of-interest based on a morphological image including a portion to be evaluated of a subject, the morphological image being acquired by an image acquisition section, and the curved surface being a surface on which an anterior-posterior position of the subject changes in a cranial-caudal direction and in a left-right direction along a position of a nerve to be evaluated; and
an estimation section configured to estimate current distribution in the region-of-interest based on magnetic data, the magnetic data being acquired by measuring a magnetic field with a magnetic measurement section, and the magnetic field being generated by electrical activity of a nerve in the portion to be evaluated.

2. The biomagnetism measurement apparatus according to claim 1, further comprising:
a display section configured to display the morphological image; and
an operation section configured to receive an anatomical characteristic position that is an indicator of the position of the nerve to be evaluated, the nerve to be evaluated being input based on the morphological image displayed on the display section, and
wherein the region-of-interest setting section sets the region-of-interest based on the position of the nerve to be evaluated received by the operation section.

3. The biomagnetism measurement apparatus according to claim 1 or 2, further comprising an extraction section configured to extract an anatomical characteristic position that is an indicator of the position of the nerve to be evaluated from the morphological image, and
wherein the region-of-interest setting section sets the region-of-interest based on the anatomical characteristic position extracted by the extraction unit.

4. The biomagnetism measurement apparatus according to any one of claims 1 to 3, wherein the nerve to be evaluated includes a nerve of a cervical spinal cord and a nerve around cervical spine.

5. The biomagnetism measurement apparatus according to claim 4, wherein the anatomical characteristic position that is an indicator of the position of the nerve to be evaluated includes a position 2 cm to 4 cm from a medial of a coracoid process to a center side and a depth position of a center of an anterior margin of a seventh cervical vertebra.

6. The biomagnetism measurement apparatus according to any one of claims 1 to 5, wherein the nerve to be evaluated includes a nerve of a lumbar cord and a nerve around lumbar spine.

7. The biomagnetism measurement apparatus according to claim 6, wherein the anatomical characteristic position that is an indicator of the position of the nerve to be evaluated includes a position of a greater sciatic notch and a depth position of an intersection of a posterior margin of a first sacral vertebra and an inferior margin of a pedicle.

8. A biomagnetism measurement system comprising:
an image acquisition section configured to acquire a morphological image including a portion to be evaluated of a subject;
a magnetic measurement section configured to measure biomagnetism; and
a biomagnetism measurement apparatus including:
a region-of-interest setting section configured to set a curved surface as a region-of-interest based on the morphological image acquired by the image acquisition section, and the curved surface being a surface on which an anterior-posterior position of the subject changes in a cranial-caudal direction and in a left-right direction along a position of a nerve to be evaluated; and
an estimation section configured to estimate current distribution in the region-of-interest based on magnetic data, the magnetic data being acquired by measuring a magnetic field with the magnetic measurement section, and the magnetic field being generated by electrical activity of a nerve in the portion to be evaluated.

9. A biomagnetism measurement system comprising a biomagnetism measurement apparatus,
wherein the biomagnetism measurement apparatus includes:
a virtual electrode setting section configured to set a virtual electrode in a three-dimensional space according to a change in a position of a nerve to be evaluated in the three-dimensional space, based on a morphological image including a portion to be evaluated of a subject, and the morphological image being acquired by an image acquisition section; and
a current waveform acquisition section configured to acquire a current waveform estimated by the virtual electrode set by the virtual electrode setting section.

10. A biomagnetism measurement method comprising:
setting a curved surface as a region-of-interest based on a morphological image including a portion to be evaluated of a subject, the morphological image being acquired by an image acquisition section, and the curved surface being a surface on which an anterior-posterior position of the subject changes in a cranial-caudal direction and in a left-right direction along a position of a nerve to be evaluated; and
estimating current distribution in the region-of-interest based on magnetic data, the magnetic data being acquired by measuring a magnetic field with a magnetic measurement section, and the magnetic field being generated by electrical activity of a nerve in the portion to be evaluated.

11. A non-transitory computer-readable recording medium storing a biomagnetism measurement program that causes a computer to execute a process, the process comprising:
setting a curved surface as a region-of-interest based on a morphological image including a portion to be evaluated of a subject, the morphological image being acquired by an image acquisition section, and the curved surface being a surface on which an anterior-posterior position of the subject changes in a cranial-caudal direction and in a left-right direction along a position of a nerve to be evaluated; and
estimating current distribution in the region-of-interest based on magnetic data, the magnetic data being acquired by measuring a magnetic field with a magnetic measurement section, and the magnetic field being generated by electrical activity of a nerve in the portion to be evaluated.
